# EUROPEAN PATENT SPECIFICATION

(11) **EP 1 931 430 B1**
(45) Date of publication and mention of the grant of the patent: **22.11.2017**
(21) Application number: 06814435.1
(22) Date of filing: 12.09.2006
(51) Int. Cl.: A61Q 11/00, A61K 8/38, A61K 8/22, A61K 8/20, A61K 8/86, A61K 8/89, A61K 8/73, A61K 8/25, A61K 8/21, A61K 8/24, A61K 8/85

(54) **DUAL PHASE WHITENING DENTIFRICE**
ZWEIPHASEN-WEISSMACHER-ZAHNCREME
DENTIFRICE BLANCHISSANT DOUBLE PHASE

(30) Priority: 27.09.2005 US 236094
(43) Date of publication of application: 18.06.2008
(73) Proprietor: Colgate-Palmolive Company, New York, NY 10022 (US)
(72) Inventor: WANG, Qin, New Jersey 08852 (US); CHOPRA, Suman, K., NJ 08831 (US); ZAIDEL, Lynette, New Jersey 07016 (US); PRENCIPE, Michael, NJ 08550 (US)
(74) Representative: Wibbelmann, Jobst
(86) International application number: PCT/US2006/035277
(87) International publication number: WO 2007/037960

(56) References cited:
- EP-A2- 0 202 359
- EP-A2- 0 867 173
- WO-A-97/21419
- WO-A-98/20849
- WO-A-98/22079
- WO-A-99/17734
- WO-A1-2005/016299
- WO-A2-2006/071675
- US-A- 3 480 557
- US-A- 5 401 495
- US-A- 5 846 570
- US-A1- 2003 091 516
- US-A1- 2005 163 729
- US-B1- 6 290 935

## Description

### BACKGROUND OF THE INVENTION

Many individuals desire a "bright" smile and white teeth, and consider dull and stained teeth cosmetically unattractive. Unfortunately, without preventive or remedial measures, stained teeth are almost inevitable due to the absorbent nature of dental material. Everyday activities such as smoking or other oral use of tobacco products, and eating, chewing or drinking certain foods and beverages (in particular coffee, tea and red wine), cause undesirable staining of surfaces of teeth. Staining can also result from microbial activity, including that associated with dental plaque. The chromogens or color causing substances in these materials become part of the pellicle layer and can permeate the enamel layer. Even with regular brushing and flossing, years of chromogen accumulation can impart noticeable tooth discoloration.

There are a variety of compositions described in the art for preventing or treating the discoloration of teeth. In particular, to combat staining and brighten or restore the natural enamel color, a variety of products containing bleaching materials are commercially available for professional and consumer use. The materials most commonly used in teeth whitening today are peroxides. Such peroxides include hydrogen peroxide, carbamide peroxide, sodium perborate, and sodium percarbonate. When these peroxides are in appropriate contact with teeth they will usually oxidize the majority of stains, rendering the teeth whiter.

Current home whitening treatment methods include abrasive toothpastes, toothpastes that produce oxides, whitening gels for use with a dental tray and whitening strips. The effectiveness of such techniques depends on a variety of factors including the type and intensity of the stain, the type of bleaching agent, contact time of the bleaching agent on the teeth, the amount of available bleaching active in the composition, the ability of the bleaching agent to penetrate the tooth enamel, and consumer compliance.

It would be desirable to provide oral care compositions having enhanced whitening effects and superior cleaning abilities.
WO 98/22079 A discloses a dentifrice composition which may provide an improved anti-tartar activity. The dentifrice composition may be a two-phase composition including a peroxide selected from the group consisting of hydrogen peroxide, calcium peroxide, urea peroxide, and mixtures thereof.

### BRIEF SUMMARY OF THE INVENTION

The invention provides a dual phase whitening oral care composition as set forth in the claims.

### DETAILED DESCRIPTION OF THE INVENTION

The present invention provides oral care compositions wherein separating the whitening agent of the first phase from the
abrasive and tartar control system in the second phase allows for delivery of a highly efficacious whitening and cleaning oral care product that is shelf-stable.

The first phase comprises a whitening agent and a substantially anhydrous carrier. The total concentration of water in the first phase, including any free water and all water contained in any ingredients, is less than 10% water by weight. This contributes to the stabilization of the whitening agent.

The first phase can optionally comprise at least one orally acceptable source of fluoride ions. Suitable sources of fluoride ions include fluoride, monofluorophosphate and fluorosilicate salts. Any such salt that is orally acceptable can be used, including without limitation alkali metal (*e.g*., potassium, sodium), ammonium, stannous and indium salts and the like. Water-soluble fluoride-releasing salts are typically used. Amine fluorides, including olaflur (N'-octadecyltrimethylendiamine-N,N,N'-tris(2-ethanol)-dihydrofluoride) may also be used. One or more fluoride-releasing salts are optionally present in an amount providing a total of about 100 to about 20,000 ppm, about 200 to about 5,000 ppm, or about 500 to about 2,500 ppm, fluoride ions. Where sodium fluoride is the sole fluoride-releasing salt present, it is preferably present at a level of from about 0.01% to about 5%, from about 0.05% to about 1%, or from about 0.1% to about 0.5%.

The first phase carrier is a low water content orally acceptable carrier. As used herein, an "orally acceptable carrier" refers to a material or combination of materials that are safe for use in the compositions of the present invention, commensurate with a reasonable benefit/risk ratio, with which the whitening agent, abrasive, and anticalculus agents (in the separate first and second phases and/or as mixed) may be associated while retaining significant efficacy. Preferably, the carrier does not substantially reduce the efficacy of the active materials of the present compositions.

The first phase carrier may also comprise various dentifrice ingredients to adjust the rheology and feel of the composition such as humectants, surface active agents, thickening or gelling agents, etc. It is preferred that the combination of ingredients are acidic to maintain stability of the whitening agent. Thus, the pH of the first phase is from 4 to 6. The first phase carrier is a gel comprising polyethylene glycol. Other suitable materials include PEG 400 MW, PEG 600 MW, and polymers and copolymers of PEG, of ethylene oxide, and of propylene oxide, for example, PLURAFLO® L4370 and/or L1220, each sold by BASF, Wyandotte, Michigan, United States of America.

The first phase preferably comprises a surface active agent. In various embodiments, suitable surface active agents may function as a surface active agent, emulsifier, and/or foam modulator. Surface active agents generally achieve increased prophylactic action, by thoroughly dispersing the whitening agent throughout the oral cavity. Any orally acceptable surfactant, most of which are anionic, nonionic or amphoteric, can be used. Suitable anionic surfactants include without limitation water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀ aliphatic secondary and tertiary amines having an anionic group such as carboxylate, sulfate, sulfonate, phosphate or phosphonate. A suitable example is cocoamidopropyl betaine.

The first phase optionally comprises a thickener. Thickeners, or gelling agents, may be selected from the group consisting of silicone fluids, carbomers, natural and synthetic gums, colloids, and mixtures thereof. In a still further embodiment a composition of the invention comprises at least one thickening agent, useful for example to impart a desired rheology, consistency, and/or mouth feel to the composition. Any orally acceptable thickening agent can be used, including without limitation carbomers, also known as carboxyvinyl polymers, carrageenans, also known as Irish moss and more particularly 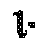 -carrageenan (iota-carrageenan), cellulosic polymers such as hydroxyethylcellulose, carboxymethylcellulose (CMC) and salts thereof, *e.g.,* CMC sodium, natural gums such as karaya, xanthan, gum arabic and tragacanth, colloidal magnesium aluminum silicate, colloidal silica and the like. One or more thickening agents are optionally present in a total amount of about 0.1% to about 90%, for example about 1% to about 50% or about 5% to about 35% by weight of the first phase.

The first phase carrier comprises a mixture of polyethylene glycol, ethylene oxide propylene oxide copolymer, and silicone. The combination provides a first phase having a desirable viscosity that is temperature stable.

Any orally acceptable pH modifying agent can be included in the carrier, including carboxylic, phosphoric, and sulfonic acids, acid salts (*e.g.,* monosodium citrate, disodium citrate, monosodium malate, etc.), alkali metal hydroxides such as sodium hydroxide, carbonates such as sodium carbonate, bicarbonates, sesquicarbonates, borates, silicates, phosphates (*e.g.,* monosodium phosphate, trisodium phosphate, pyrophosphate salts, etc.), imidazole, and mixtures thereof. One or more pH modifying agents are optionally present in a total amount effective to maintain the composition in an orally acceptable pH range.

The second phase comprises an abrasive and an anticalculus agent in an orally acceptable carrier. Without limiting the mechanism, function or utility of present invention, it is believed that the combination of active ingredients in the second phase and the pH difference between the first and second phases assist in improved whitening efficacy and whitening agent release.

The dentally acceptable abrasive material or polishing agent may serve to either polish the tooth enamel or provide or enhance the whitening effect of the composition. Any orally acceptable abrasive can be used. Suitable abrasives include without limitation silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, β-calcium pyrophosphate, tricalcium phosphate, calcium polymetaphosphate and insoluble sodium polymetaphosphate. A preferred abrasive is a high cleaning silica abrasive. One or more abrasives are optionally present in an abrasive effective total amount, typically from about 0.1% to about 40% by weight of the second phase. Average particle size of an abrasive, if present, is generally about 0.1 to about 30 µm, for example about 1 to about 20 µm or about 5 to about 15 µm.

The oral composition contains an anticalculus agent. One or more such agents can be present. Suitable anticalculus agents include any known or to be developed in the art, such as phosphates and polyphosphates (for example pyrophosphates), polyaminopropanesulfonic acid (AMPS), hexametaphosphate salts, zinc citrate trihydrate, polypeptides such as polyaspartic and polyglutamic acids, polyolefin sulfonates, polyolefin phosphates, diphosphonates such as azacycloalkane-2,2-diphosphonates (*e.g*., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids, salts of any of these agents, for example their alkali metal and ammonium salts, and mixtures thereof.

The second phase may optionally include a peroxide activator. Peroxide activators of the present invention are preferably transition metal catalysts, alkaline compounds, or mixtures thereof. The peroxide activators accelerate the whitening effect of the composition and provide high efficacy using lower concentrations of the peroxygen compound.

If desired, a transition metal catalyst can comprise any of the stable transition elements in Groups 3 through 12 of the periodic table including cadmium, chromium, cobalt, copper, gold, hafnium, iridium, iron, lutetium, manganese, mercury, molybdenum, nickel, niobium, osmium, palladium, platinum, rhenium, rhodium, ruthenium, scandium, silver, tantalum, titanium, tungsten, vanadium, yttrium, zinc, zirconium, and combinations thereof. In particular, the transition metal catalyst can comprise iron, cobalt, nickel, copper, zinc, manganese, chromium, and combinations thereof. A preferred transition metal catalyst is manganese.

In various embodiments, the orally acceptable vehicle used to prepare the second phase of the oral care composition is a gel or paste. The humectants, surface active agents, and thickeners, as described above may be used in the second phase carrier.

Preferably, the second phase carrier also includes water. Water employed should preferably be deionized and free of organic impurities. The water is free water which is added, plus that which is introduced with other materials for example, such as that added with sorbitol. Water generally comprises from about 10% to 50%, preferably from about 20% to 40%, by weight of the second phase. The second phase carrier may also include fluoride as described above.

It is understood that the inclusion of certain ingredients may be altered depending on the pH of the respective phase and/or any potential side interactions with the active ingredients in the first and second phase as known to one of skill in the art.

As recognized by one of skill in the art, the oral compositions (both the first and/or second phases) of the present invention optionally include other materials, such as for example, anti-caries agents; desensitizing agents; viscosity modifiers; diluents; surface active agents, such as surfactants, emulsifiers, and foam modulators; pH modifying agents; abrasives, in addition to those listed above herein; humectants; mouth feel agents; sweetening agents; flavor agents; foam modulators, active agents (including pharmaceutical agents, topical or systematic agents) colorants; preservatives; and combinations thereof.

Methods are provided to whiten a tooth surface in a human or animal subject comprising maintaining a whitening oral care composition as claimed having a first phase comprising a whitening agent and a substantially anhydrous and orally acceptable carrier; and a second phase comprising an abrasive and a tartar control system in an orally acceptable carrier, where the first and second phases are separated from one another; mixing the first phase and the second phase; and contacting the mixed composition with the tooth surface. As used herein "animal subject" includes higher order non-human mammals such as canines, felines, and horses. The oral care composition is contacted with a tooth surface of the mammalian subject to thereby whiten teeth in a highly efficacious manner, without any negative interaction between the whitening agent, tartar control agent, and abrasive ingredients.

In various embodiments, it is preferred that the oral care composition is applied and contacted with the tooth surface. The dentifrice, prepared in accordance with the present invention is preferably applied regularly to a tooth surface, preferably on a daily basis, at least one time daily for multiple days, but alternately every second or third day. Preferably the oral composition is applied to the tooth surfaces from 1 to 3 times daily, at a pH of greater than about 7, preferably from about pH 8 to 10, for at least 2 weeks up to 8 weeks, from four months to three years, or more up to lifetime.

The compositions of the present invention may be packaged in any of a variety of packages, including dual compartment containers among those known in the art. Preferably, such packages contain the first phase and second phase so that the two phases are not in substantial contact until dispensing during use. In various embodiments, the first phase is stored in a first enclosure; the second phase is stored in a second enclosure; and the first phase is expelled from the first enclosure and the second phase is expelled from the second enclosure just prior to application to the teeth so that the first phase and the second phase are expelled to provide an amalgam comprising a first phase in fluid interface with a second phase. This embodiment is preferably provided to the consumer in the form of an oral care kit or package providing (a) a first chamber (the first storage enclosure for the first phase) having a first outlet in fluid communication with the first chamber for discharge of the first phase; and (b) a second chamber (the second storage enclosure for the second phase) having a second outlet in fluid communication with the second chamber for discharge of the second phase. The second outlet is proximate to the first outlet so that, the first and second phases are discharged substantially simultaneously. Such a package is also denoted herein as a dual compartment toothpaste tube. In some embodiments, approximately equal amounts of each phase are delivered into the amalgam so that the consumer has a convenient basis for ascertaining that both phases are being delivered and that rapid intermixing of the phases will occur as the amalgam is brushed against the teeth. In some embodiments, dissimilar amounts of each phase are delivered.

The invention is illustrated in the following non-limiting examples.

### Example 1

**Table 1**

| Ingredients | 1^{st} Phase | 2^{nd} Phase |
|---|---|---|
| Ethylene oxide and Propylene oxide block copolymer (PLURAFLO® L4370) | 43.557 | |
| Cross-linked PVP hydrogen peroxide complex | 22.0 | |
| Sodium fluoride | 0.243 | |
| Silicone fluid 350 CST | 5.0 | |
| Ethylene oxide and Propylene oxide block copolymer (PLURAFLO® L1220) | 25.0 | |
| Fumed silica A200 | 2.0 | |
| Flavor | 1.2 | |
| Sodium saccharin | 1.0 | |
| Synthetic glycerin | | 12.0 |
| Sorbitol - non browning/non crystallizing | | 27.5 |
| Purified water | | 7.98 |
| Hydrated silica (SYLODENT® 783) | | 11.0 |
| Hydrated silica (SYLODENT® XWA 650) | | 10.0 |
| Hydrated silica (ZEODENT® 165) | | 1.7 |
| Tetra sodium pyrophosphate | | 1.0 |
| Sodium tripolyphosphate | | 7.0 |
| Sodium carboxy methylcellulose 2000S | | 0.95 |
| Iota carrageenan | | 0.35 |
| Laponite D | | 0.75 |
| SO₃ sodium lauryl sulfate 29% | | 7.33 |
| Flavor | | 1.15 |
| Sodium saccharin | | 0.55 |
| Titanium dioxide | | 1.0 |
| Vinyl methyl ether (GANTREZ®) | | 7.69 |
| Sodium hydroxide - 50% solution | | 2.0 |
| Manganese gluconate anhydrous | | 0.05 |
| TOTAL | 100 | 100 |

A dual phase dentifrice is prepared according to Table 1. The first phase whitening agent is a cross-linked polyvinyl pyrrolidone hydrogen peroxide complex whitening agent. The first phase carrier includes polyethylene glycol, a copolymer of ethylene oxide and propylene oxide, and a silicone fluid. The first phase carrier does not include any free water and has a proper pH to facilitate delivery of a stable PVP-hydrogen peroxide complex. Fluoride is included in the first phase to enhance oral care benefits of the dentifrice, particularly anti-caries benefits. The second phase includes the anti-calculus agents tetrasodium pyrophosphate, sodium tripolyphosphate, and vinyl methyl ether (GANTREZ®-97). The pH of the second phase is raised to a sufficiently high amount with the sodium hydroxide solution. The second phase also includes a manganese gluconate activator agent. The dual phase dentifrice provides superior cleaning and whitening benefits.

### Example 2

A dual phase dentifrice is prepared according to Example 1. The dentifrice is stored in a dual chamber container where the first phase is separated from the second phase. The dentifrice is dispensed onto a tooth brush where the contents of the first and second phase are initially mixed together. A subject begins to brush their teeth with the dentifrice and the shear force further mixes the two phases. The oxidizing activity of the hydrogen peroxide whitening agent is released throughout mixing of the phases and provides enhanced whitening.

## Claims

1. A dual phase whitening oral care composition, comprising:
a. a first phase having a pH of 4 to 6 and comprising a cross-linked polyvinyl pyrrolidone hydrogen peroxide complex whitening agent in a substantially anhydrous and orally acceptable carrier, said carrier comprising a mixture of polyethylene glycol, ethylene oxide propylene oxide copolymer, and silicone, wherein the water content of the first phase is less than 10% by weight of the first phase; and
b. a second phase having a different pH than the first phase and comprising an abrasive and an anticalculus agent in an orally acceptable carrier; wherein the first phase and the second phase are maintained separately from each other until dispensed.

2. A dual phase whitening oral care composition according to claim 1, wherein the first phase comprises the whitening agent at a level of from 0.1% to 30% by weight of the first phase.

3. A dual phase whitening oral care composition according to claim 1, wherein the substantially anhydrous carrier further comprises at least one of a surfactant and a thickener selected from the group consisting of silicone fluids, fumed silica, polyethylene glycol, carbomers, and gums.

4. A dual phase whitening oral care composition according to claim 1, wherein the second phase further comprises at least one agent selected from the group consisting of a pH adjusting agent, and a fluoride providing agent.

5. A dual phase whitening oral care composition according to claim 1, wherein the abrasive of the second phase is a silica abrasive.

6. A dual phase whitening oral care composition according to claim 1, wherein the anticalculus agent of the second phase is selected from the group consisting of: inorganic phosphate salts, inorganic polyphosphate salts, polymeric polycarboxylates, sequestering agents, and mixtures thereof.

7. A dual phase whitening oral care composition according to claim 1, wherein the second phase further comprises a peroxide activator selected from the group consisting of transition metal catalysts, alkaline compounds, and combinations thereof.

## Patentansprüche

1. Zweiphasenaufhellungs-Mundpflegezusammensetzung, umfassend:
a. eine erste Phase mit einem pH-Wert von 4 bis 6, umfassend einen quervernetzten Polyvinylpyrrolidon-Wasserstoffperoxid-Komplex als Aufhellmittel in einem im Wesentlichen wasserfreien und oral annehmbaren Träger, wobei der Träger eine Mischung aus Polyethylen-Glykol, Ethylenoxid Propylenoxid Copolymer und Silikon umfasst, wobei der Wassergehalt der ersten Phase weniger als 10 Gewichtsprozent der ersten Phase ist; und
b. eine zweite Phase mit einem anderem pH-Wert als die erste Phase und umfassend eine Abrasivstoff und ein Anticalculusmittel in einem oral einnehmbaren Träger, wobei die erste und die zweite Phase getrennt voneinander gehalten werden bis sie abgegeben werden.

2. Zweiphasenaufhellungs-Mundpflegezusammensetzung nach Anspruch 1, worin die erste Phase ein Aufhellungsmittel bei einer Konzentration von 0,1 bis 30 Gewichtsprozent der ersten Phase umfasst.

3. Zweiphasenaufhellungs-Mundpflegezusammensetzung nach Anspruch 1, worin der im Wesentlichen wasserfreie Träger weiterhin mindestens ein Tensid umfasst und ein Verdickungsmittel ausgewählt aus der Gruppe bestehend aus Silikonflüssigkeiten, pyrogene Kieselsäure, Polyethylenglykol, Carbomere und Gummen.

4. Zweiphasenaufhellungs-Mundpflegezusammensetzung nach Anspruch 1, worin die zweite Phase mindestens ein Mittel umfasst ausgewählt aus der Gruppe bestehend aus einem pH-Wert Einstellmittel und einem fluoridbereitstellenden Mittel.

5. Zweiphasenaufhellungs-Mundpflegezusammensetzung nach Anspruch 1, worin der Abrasivstoff der zweiten Phase ein Silica-Abrasivstoff ist.

6. Zweiphasenaufhellungs-Mundpflegezusammensetzung nach Anspruch 1, worin das Anticaculusmittel der zweiten Phase ausgewählt ist aus der Gruppe bestehend aus: anorganische Phosphatsalze, anorganische Polyphosphatsalzen, polymeren Polycarboxylaten, Komplexierungsmitteln und Mischungen davon.

7. Zweiphasenaufhellungs-Mundpflegezusammensetzung nach Anspruch 1, worin die zweite Phase weiterhin umfasst, einen Peroxid-Aktivator ausgewählt aus der Gruppe bestehend aus Übergangsmetallkatalysatoren, alkalischen Verbindungen und Kombinationen davon.

## Revendications

1. Composition de soin buccal blanchissante à deux phases comprenant :
a. une première phase ayant un pH de 4 à 6 et comprenant un agent de blanchiment complexe de peroxyde d'hydrogène et de polyvinyl pyrrolidone réticulée dans un support sensiblement anhydre et acceptable par voie orale, ledit support comprenant un mélange de polyéthylène glycol, de copolymère d'oxyde d'éthylène et d'oxyde de propylène, et de silicone, dans laquelle la teneur en eau de la première phase est inférieure à 10 % en poids de la première phase ; et
b. une seconde phase ayant un pH différent de la première phase et comprenant un abrasif et un agent antitartre dans un support acceptable par voie orale ; dans laquelle la première phase et la seconde phase sont maintenues séparément l'une de l'autre jusqu'à distribution.

2. Composition de soin buccal blanchissante à deux phases selon la revendication 1, dans laquelle la première phase comprend l'agent de blanchiment à un taux de 0,1 % à 30 % en poids de la première phase.

3. Composition de soin buccal blanchissante à deux phases selon la revendication 1, dans laquelle le support sensiblement anhydre comprend en outre au moins un élément parmi un tensioactif et un agent épaississant choisi dans le groupe constitué par les fluides de silicone, la silice fumée, le polyéthylène glycol, les carbomères et les gommes.

4. Composition de soin buccal blanchissante à deux phases selon la revendication 1, dans laquelle la seconde phase comprend en outre au moins un agent choisi dans le groupe constitué par un agent d'ajustement du pH et un agent de fourniture de fluorure.

5. Composition de soin buccal blanchissante à deux phases selon la revendication 1, dans laquelle l'abrasif de la seconde phase est un abrasif à base de silice.

6. Composition de soin buccal blanchissante à deux phases selon la revendication 1, dans laquelle l'agent antitartre de la seconde phase est choisi dans le groupe constitué par : les sels de phosphate inorganiques, les sels de polyphosphate inorganiques, les polycarboxylates polymères, les agents séquestrants et les mélanges de ceux-ci.

7. Composition de soin buccal blanchissante à deux phases selon la revendication 1, dans laquelle la seconde phase comprend en outre un activateur de peroxyde choisi dans le groupe constitué par les catalyseurs à métaux de transition, les composés alcalins et les combinaisons de ceux-ci.
